# EUROPÄISCHE PATENTSCHRIFT

(11) **EP 1 087 946 B1**
(45) Veröffentlichungstag und Bekanntmachung des Hinweises auf die Patenterteilung: **10.01.2007**
(21) Anmeldenummer: 99939760.7
(22) Anmeldetag: 07.05.1999
(51) Int. Cl.: C07D 237/04, C07D 405/04, A61K 31/50

(54) **ARYLALKANOYLPYRIDAZINE**
ARYL ALKANOYLPYRIDAZINES
ARYLALCANOYLPYRIDAZINES

(30) Priorität: 16.06.1998 DE 19826841
(43) Veröffentlichungstag der Anmeldung: 04.04.2001
(73) Patentinhaber: Merck Patent GmbH, 64293 Darmstadt (DE)
(72) Erfinder: JONAS, Rochus, D-64291 Darmstadt (DE); WOLF, Michael, D-64297 Darmstadt (DE); KLUXEN, Franz-Werner, D-64285 Darmstadt (DE)
(74) Vertreter: Isenbruck, Günter
(86) Internationale Anmeldenummer: PCT/EP1999/003151
(87) Internationale Veröffentlichungsnummer: WO 1999/065880

(56) Entgegenhaltungen:
- DE-A- 19 632 549

## Beschreibung

Die Erfindung betrifft Arylalkanoylpyridazinderivate der Formel I 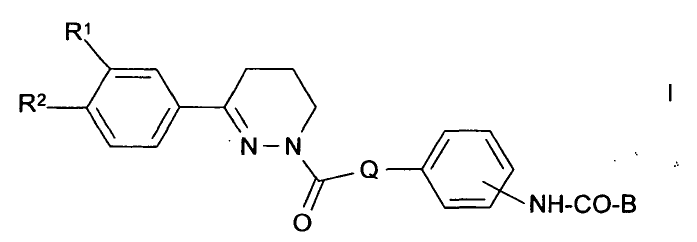 worin
- B: o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-N,N-Dimethylaminophenyl, o-, m- oder p-Nitrophenyl, o-, m-oder p-Hydroxyphenyl, o-, m- oder p-Methoxyphenyl, o-, m- oder p-Ethoxyphenyl, o-, m- oder p-Isopropoxyphenyl, o-, m- oder p-Butoxyphenyl, o-, m- oder p-Pentoxyphenyl, o-, m- oder p-Hexyloxyphenyl, o-, m- oder p-Decyloxyphenyl, o-, m-, p-Trifluormethylphenyl, o-, m- oder p-Fluorphenyl, o-, m- oder p-Chlorphenyl, o-, m- oder p-Bromphenyl, o-, m- oder p-Acetylaminophenyl, o-, m- oder p-Isopropylcarbonylaminophenyl, o-, m-oder p-Methansulfonylaminophenyl, o-, m- oder p-Ethansulfonylaminophenyl, o-, m- oder p-Methoxycarbonyl-aminophenyl, o-, m- oder p-Ethoxycarbonyl-aminophenyl, 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dimethoxyphenyl,
- Q: fehlt,
- R¹, R²: jeweils unabhängig voneinander -OR⁴,
- R⁴: A, Cycloalkyl mit 3-7 C-Atomen,
- A: Alkyl mit 1 bis 10 C-Atomen, das durch 1 bis 5 F- und/oder Cl-Atome substituiert sein kann und

- Hal: F, Cl, Br oder I
bedeuten,
sowie deren physiologisch unbedenklichen Salze und Solvate.

1-Benzoyl-tetrahydropyridazine als Progesteron-Rezeptorliganden sind z.B. in J. Med.Chem. 38, 4878 (1995) beschrieben.
Ähnliche Verbindungen sind auch aus DE 196 32 549 A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und Solvate bei guter Verträglichkeit sehr wertvolle pharmakologische Eigenschaften besitzen.

Insbesondere zeigen sie eine selektive Phosphodiesterase IV-Hemmung, die mit einer intrazellulären Erhöhung von cAMP verbunden ist (N. Sommer et al., Nature Medicine, 1, 244-248 (1995)).
Die PDE IV-Hemmung kann z.B. analog C.W. Davis in Biochim. biophys. Acta **797**, 354-362 (1984) nachgewiesen werden.

Die erfindungsgemäßen Verbindungen können zur Behandlung von asthmatischen Erkrankungen eingesetzt werden. Die antiasthmatische Wirkung der PDE IV-Hemmer ist z.B. von T.J. Torphy et al. in Thorax, **46**, 512-523 (1991) beschrieben und kann z. B. nach der Methode von T. Olsson, Acta allergologica **26**, 438-447 (1971), bestimmt werden.

Da cAMP knochenabbauende Zellen hemmt und knochenaufbauende Zellen stimuliert (S. Kasugai et al., M 681 und K. Miyamoto, M 682, in Abstracts of the American Society for Bone and Mineral Research 18^{th} Annual Meeting, 1996), können die erfindungsgemäßen Verbindungen zur Behandlung von Osteoporose eingesetzt werden.

Die Verbindungen zeigen außerdem eine antagonistische Wirkung auf die Produktion von TNF (Tumor Nekrose Faktor) und eignen sich daher zur Behandlung von allergischen und entzündlichen Krankheiten, Autoimmunkrankheiten, wie z.B. rheumatoide Arthritis, multiple Sklerose, Morbus Crohn, Diabetes mellitus oder ulzerative Kolitis, Transplantatabstoßungsreaktionen, Kachexie und Sepsis.

Die antiinflammatorische Wirkung der erfindungsgemäßen Substanzen und ihre Wirksamkeit zur Behandlung von z.B. Autoimmunerkrankungen wie multipler Sklerose oder rheumatoider Arthritis, kann analog den Methoden von N. Sommer et al., Nature Medicine 1, 244-248 (1995) oder L. Sekut et al., Clin. Exp. Immunol. **100**, 126-132 (1995) bestimmt werden.

Die Verbindungen können zur Behandlung von Kachexie eingesetzt werden. Die anti-kachektische Wirkung kann in TNF-abhängigen Modellen der Kachexie geprüft werden (P. Costelli et al., J. Clin. Invest. **95**, 2367ff. (1995); J.M. Argiles et al., Med. Res. Rev. **17**, 477ff. (1997)).

PDE IV-Inhibitoren können auch das Wachstum von Tumorzellen hemmen und sind deshalb für die Tumortherapie geeignet (D. Marko et al., Cell Biochem. Biophys. **28**, 75ff. (1998)). Die Wirkung von PDE IV-Hemmern bei der Tumorbehandlung ist z.B. in der WO 95 35 281, WO 95 17 399 oder WO 96 00 215 beschrieben.

PDE IV-Inhibitoren können die Mortalität in Modellen für Sepsis verhindern und eignen sich daher für die Therapie von Sepsis (W. Fischer et al., Biochem. Pharmacol. **45**, 2399ff. (1993)).

Sie können weiterhin zur Behandlung von Gedächtnisstörungen, Atherosklerose, atopische Dermatitis und AIDS eingesetzt werden.

Die Wirkung von PDE IV-Hemmern bei der Behandlung von Asthma, entzündlichen Erkrankungen, Diabetes mellitus, atopischer Dermatitis, Psoriasis, AIDS, Kachexie, Tumorwachstum oder Tumormetastasen ist z.B. in der EP 77 92 91 beschrieben.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung von Verbindungen der Formel

I nach Anspruch 1 sowie deren Salze und Solvate, dadurch gekennzeichnet, daß man eine Verbindung der Formel II 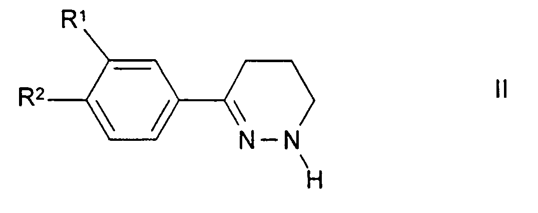 worin
R¹ und R² die angegebenen Bedeutungen haben,
mit einer Verbindung der Formel III 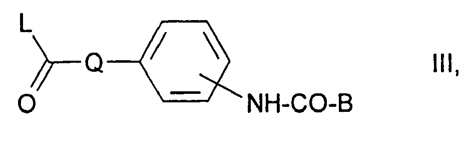 worin
- B und Q: die angegebenen Bedeutungen haben und

- L: Cl, Br, OH oder eine reaktionsfähige veresterte OH-Gruppe bedeutet,
umsetzt,
oder
daß man eine Verbindung der Formel IV 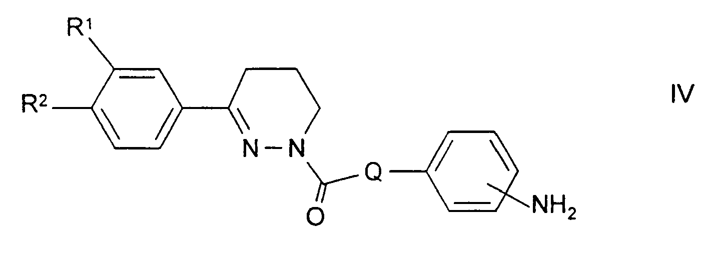 worin
R¹, R² und Q die angegebenen Bedeutungen haben, mit einer Verbindung der Formel V

B-CO-L V

worin
- B: die angegebene Bedeutung hat, und
- L: Cl, Br, OH oder eine reaktionsfähige veresterte OH-Gruppe bedeutet,
umsetzt,
und/oder daß man eine basische Verbindung der Formel I durch Behandeln mit einer Säure in eines ihrer Salze überführt.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B: Mono- oder Dihydrate oder Alkoholate.

Vor- und nachstehend haben die Reste R¹, R², B, Q und L die bei den Formeln I, II, III, IV und V angegebenen Bedeutungen, sofern nicht ausdrücklich etwas anderes angegeben ist.

A bedeutet vorzugsweise Alkyl, weiter bevorzugt durch 1 bis 5 Fluor- und/oder Chloratome substituiertes Alkyl.

In den vorstehenden Formeln ist Alkyl vorzugsweise unverzweigt und hat 1,2,3, 4, 5, 6, 7, 8, 9 oder 10 C-Atome, vorzugsweise 1, 2, 3, 4, 5 oder 6 C-Atome und bedeutet vorzugsweise Methyl, Ethyl, Trifluormethyl, Pentafluorethyl oder Propyl, weiterhin bevorzugt Isopropyl, Butyl, Isobutyl, sek.-Butyl oder tert.-Butyl, aber auch n-Pentyl, neo-Pentyl, Isopentyl oder n-Hexyl. Besonders bevorzugt ist Methyl, Ethyl, Trifluormethyl, Propyl, Isopropyl, Butyl, n-Pentyl, n-Hexyl oder n-Decyl.

Cycloalkyl hat vorzugsweise 3-7 C-Atome und steht bevorzugt für Cyclopropyl und Cyclobutyl, weiterhin bevorzugt für Cyclopentyl oder Cyclohexyl, ferner auch für Cycloheptyl, besonders bevorzugt ist Cyclopentyl.

Alkenyl steht vorzugsweise für Allyl, 2- oder 3-Butenyl, Isobutenyl, sek.-Butenyl, ferner bevorzugt ist 4-Pentenyl, iso-Pentenyl oder 5-Hexenyl.

Alkylen ist vorzugsweise unverzweigt und bedeutet bevorzugt Methylen oder Ethylen, ferner bevorzugt Propylen oder Butylen.

Alkylencycloalkyl hat vorzugsweise 5-10 C-Atome und steht bevorzugt für Methylencyclopropyl, Methylencyclobutyl, weiterhin bevorzugt für Methylencyclopentyl, Methylencyclohexyl oder Methylencycloheptyl, ferner auch für Ethylencyclopropyl, Ethylencyclobutyl, Ethylencyclopentyl, Ethylencyclohexyl oder Ethylencycloheptyl, Propylencyclopentyl, Propylencyclohexyl, Butylencyclopentyl oder Butylencyclohexyl.

Hal bedeutet vorzugsweise F, Cl oder Br, aber auch I.

Die Reste R¹ und R² können gleich oder verschieden sein und stehen in der 3- oder 4-Position des Phenylrings. Bevorzugt stehen sie aber jeweils für Methoxy, Ethoxy, Propoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

R¹ steht besonders bevorzugt für Methoxy, Ethoxy, Cyclopentoxy oder Isopropoxy. R² steht besonders bevorzugt für Methoxy oder Ethoxy.

Besonders bevorzugt für R⁴ ist Alkyl oder Cycloalkyl mit den zuvor angegebenen Bedeutungen.

B bedeutet o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-N,N-Dimethylaminophenyl, o-, m- oder p-Nitrophenyl, o-, m- oder p-Hydroxyphenyl, o-, m- oder p-Methoxyphenyl, o-, m- oder p-Ethoxyphenyl, o-, m- oder p-Isopropoxyphenyl, o-, m- oder p-Butoxyphenyl, o-, m- oder p-Pentoxyphenyl, o-, m- oder p-Hexyloxyphenyl, o-, m- oder p-Decyloxyphenyl, o-, m-, p-Trifluormethylphenyl, o-, m- oder p-Fluorphenyl, o-, m- oder p-Chlorphenyl, o-, m- oder p-Bromphenyl, o-, m- oder p-Acetylaminophenyl, o-, m- oder p-Isopropylcarbonylaminophenyl, o-, m- oder p-Methansulfonylaminophenyl, o-, m- oder p-Ethansulfonyl-aminophenyl, o-, m- oder p-Methoxycarbonyl-aminophenyl, o-, m- oder p-Ethoxycarbonyl-aminophenyl, 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dimethoxyphenyl.

Der Rest Q fehlt.

Für die gesamte Erfindung gilt, dass sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgende Teilformel Ia ausgedrückt werden, die der Formel I entsprechen und worin die nicht näher bezeichneten Reste die bei der Formel I angegebene Bedeutung haben, worin jedoch
- in la R¹ und R²: jeweils unabhängig voneinander OA,
- Q: fehlt und
- B: die angegebene Bedeutung hat
bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II bis IV haben R¹ und R² die angege-benen Bedeutungen, insbesondere die angegebenen bevorzugten Bedeutungen.

In den Verbindungen der Formeln III und lV fehlt Q.

B hat in den Verbindungen der Formeln III und V die angegebenen bevorzugten Bedeutungen, während L Cl, Br, OH oder eine reaktionsfähige veresterte OH-Gruppe bedeutet.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt. Sofern sie nicht bekannt sind, können sie nach an sich bekannten Methoden hergestellt werden.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Verbindungen der Formel I können weiterhin erhalten werden, indem man Verbindungen der Formel IV mit Verbindungen der Formel V umsetzt.
Die Ausgangsverbindungen der Formeln IV und V sind in der Regel bekannt. Die Verbindungen der Formel IV sind z.B. aus DE 196 32 549 bekannt. Sind sie nicht bekannt, so können sie nach an sich bekannten Methoden hergestellt werden.
So ist z.B. die Herstellung von 1-Benzoyl-tetrahydropyridazin in J. Med. Chem. 38, 4878 (1995) beschrieben.

In den Verbindungen der Formel V bedeutet der Rest -CO-L eine voraktivierte Carbonsäure, vorzugsweise ein Carbonsäurehalogenid.

Die Umsetzung der Verbindungen der Formel IV mit Verbindungen der Formel V erfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthatin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können, falls gewünscht, die freien Basen der Formel I aus ihren Salzen mit Basen (z.B. Natrium- oder Kaliumhydroxid oder -carbonat) in Freiheit gesetzt werden.

Gegenstand der Erfindung sind Verbindungen der Formel I und ihre physiologisch unbedenklichen Salze und Solvate als Arzneimittel.

Gegenstand der Erfindung sind auch die Verbindungen der Formel I und ihre physiologisch unbedenklichen Salze und Solvate als Phosphodiesterase IV-Hemmer.

Gegenstand der Erfindung ist ferner die Verwendung der Verbindungen der Formel I und/oder ihrer physiologisch unbedenklichen Salze und/oder Solvate zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze und/oder Solvate.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder ein oder mehrere weitere Wirkstoffe enthalten, z.B. ein oder mehrere Vitamine.

Die Verbindungen der Formel I und ihre physiologisch unbedenklichen Salze und Solvate können bei der Bekämpfung von Krankheiten, bei denen eine Erhöhung des cAMP(cyclo-Adenosin-monophosphat)-Spiegels zu Entzündungshemmung oder -verhinderung und Muskelentspannung führt, eingesetzt werden. Besondere Verwendung können die erfindungsgemäßen PDE IV-Inhibitoren bei der Behandlung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS finden.

Dabei werden die erfindungsgemäßen Substanzen in der Regel vorzugsweise in Dosierungen entsprechend der Verbindung Rolipram zwischen 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1:

Eine Lösung von 1,1 g 1-(3-Aminobenzoyl)-3-(3-ethoxy-4-methoxyphenyl)-1,4,5,6-tetrahydro-pyridazin, F. 180° [erhältlich durch katalytische Hydrierung von 1-(3-Nitrobenzoyl)-3-(3-ethoxy-4-methoxyphenyl)-1,4,5,6-tetrahydro-pyridazin, F. 173°, in 150 ml Tetrahydrofuran in Gegenwart von 3,5 g Raney-Nickel bei Raumtemperatur] und 0,6 ml Pyridin in 50 ml Acetonitril wird mit 0,5 g 4-Chlorbenzoylchlorid versetzt und zwei Stunden nachgerührt. Man entfernt das Lösungsmittel und arbeitet wie üblich auf. Nach Umkristallisation erhält man N-(3-(3-Ethoxy-4-methoxyphenyl)-1,4,5,6-tetrahydro-pyridazin-1-ylcarbonyl)-phenyl)-4-chlorbenzoyl-3-carboxamid, F. 236°.

Analog erhält man durch Umsetzung von 1-(3-Aminobenzoyl)-3-(3-ethoxy-4-methoxyphenyl)-1,4,5,6-tetrahydro-pyridazin
mit 3-Nitrobenzoylchlorid,
N-(3-(3-Ethoxy-4-methoxyphenyl)-1,4,5,6-tetrahydro-pyridazin-1-ylcarbonyl)-phenyl)-3-nitrobenzoyl-3-carboxamid, F. 160°;
mit 4-Nitrobenzoylchlorid,
N-(3-(3-Ethoxy-4-methoxyphenyl)-1,4,5,6-tetrahydro-pyridazin-1-ylcarbonyl)-phenyl)-4-nitrobenzoyl-3-carboxamid, F. 255°;
mit 4-Methoxybenzoylchlorid,
N-(3-(3-Ethoxy-4-methoxyphenyl)-1,4,5,6-tetrahydro-pyridazin-1-ylcarbonyl)-phenyl)-4-methoxybenzoyl-3-carboxamid, F. 206°;
mit 4-Methylbenzoylchlorid,
N-(3-(3-Ethoxy-4-methoxyphenyl)-1,4,5,6-tetrahydro-pyridazin-1-ylcarbonyl)-phenyl)-4-methylbenzoyl-3-carboxamid, F. 219°;
mit Benzoylchlorid, (Vergleichsbeispiel)
N-(3-(3-Ethoxy-4-methoxyphenyl)-1,4,5,6-tetrahydro-pyridazin-1-ylcarbonyl)-phenyl)-benzoyl-3-carboxamid, F. 203°;
mit 3,4-Dichlorbenzoylchlorid,
N-(3-(3-Ethoxy-4-methoxyphenyl)-1,4,5,6-tetrahydro-pyridazin-1-ylcarbonyl)-phenyl)-3,4-dichlorbenzoyl-3-carboxamid, F. 177°;
mit 4-Trifluormethylbenzoylchlorid,
N-(3-(3-Ethoxy-4-methoxyphenyl)-1,4,5,6-tetrahydro-pyridazin-1-ylcarbonyl)-phenyl)-4-trifluormethylbenzoyl-3-carboxamid, F. 207°;
mit 3-Chlorbenzoylchlorid,
N-(3-(3-Ethoxy-4-methoxyphenyl)-1,4,5,6-tetrahydro-pyridazin-1-ylcarbonyl)-phenyl)-3-chlorbenzoyl-3-carboxamid, F. 121°;
mit 4-Fluorbenzoylchlorid,
N-(3-(3-Ethoxy-4-methoxyphenyl)-1,4,5,6-tetrahydro-pyridazin-1-ylcarbonyl)-phenyl)-4-fluorbenzoyl-3-carboxamid, F. 236°;
mit 4-Butoxybenzoylchlorid,
N-(3-(3-Ethoxy-4-methoxyphenyl)-1,4,5,6-tetrahydro-pyridazin-1-ylcarbonyl)-phenyl)-4-butoxybenzoyl-3-carboxamid, F. 123°;
mit 4-Pentoxybenzoylchlorid,
N-(3-(3-Ethoxy-4-methoxyphenyl)-1,4,5,6-tetrahydro-pyridazin-1-ylcarbonyl)-phenyl)-4-pentoxybenzoyl-3-carboxamid, F. 145°;
mit 4-Ethoxybenzoylchlorid,
N-(3-(3-Ethoxy-4-methoxyphenyl)-1,4,5,6-tetrahydro-pyridazin-1-ylcarbonyl)-phenyl)-4-ethoxybenzoyl-3-carboxamid, F. 174°;
mit 3,4-Dimethoxybenzoylchlorid,
N-(3-(3-Ethoxy-4-methoxyphenyl)-1,4,5,6-tetrahydro-pyridazin-1-ylcarbonyl)-phenyl)-3,4-dimethoxybenzoyl-3-carboxamid, F. 160°;
mit 3-Methylbenzoylchlorid,
N-(3-(3-Ethoxy-4-methoxyphenyl)-1,4,5,6-tetrahydro-pyridazin-1-ylcarbonyl)-phenyl)-3-methylbenzoyl-3-carboxamid, F. 115°;
mit 3-Methoxybenzoylchlorid,
N-(3-(3-Ethoxy-4-methoxyphenyl)-1,4,5,6-tetrahydro-pyridazin-1-ylcarbonyl)-phenyl)-3-methoxybenzoyl-3-carboxamid, F. 161°.

### Beispiel 2:

Eine Lösung von 1,1 g 1-(4-Aminobenzoyl)-3-(3-ethoxy-4-methoxyphenyl)-1,4,5,6-tetrahydro-pyridazin, F. 154° [erhältlich durch katalytische Hydrierung von 1-(4-Nitrobenzoyl)-3-(3-ethoxy-4-methoxyphenyl)-1,4,5,6-tetrahydro-pyridazin, F. 159°, in 150 ml Tetrahydrofuran in Gegenwart von 3,5 g Raney-Nickel bei Raumtemperatur] und 0,6 ml Pyridin in 50 ml Acetonitril wird mit 0,5 g 4-Nitrobenzoylchlorid versetzt und zwei Stunden nachgerührt. Man entfernt das Lösungsmittel und arbeitet wie üblich auf. Nach Umkristallisation erhält man N-(3-(3-Ethoxy-4-methoxyphenyl)-1,4,5,6-tetrahydro-pyridazin-1-ylcarbonyl)-phenyl)-4-nitrobenzoyl-4-carboxamid, F. 233°.

Analog erhält man durch Umsetzung von 1-(4-Aminobenzoyl)-3-(3-ethoxy-4-methoxyphenyl)-1,4,5,6-tetrahydro-pyridazin
mit 4-Methoxybenzoylchlorid,
N-(3-(3-Ethoxy-4-methoxyphenyl)-1,4,5,6-tetrahydro-pyridazin-1-ylcarbonyl)-phenyl)-4-methoxybenzoyl-4-carboxamid, F. 201 °;
mit 4-Fluorbenzoylchlorid,
N-(3-(3-Ethoxy-4-methoxyphenyl)-1,4,5,6-tetrahydro-pyridazin-1-ylcarbonyl)-phenyl)-4-fluorbenzoyl-4-carboxamid, F. 193°;
mit Benzoylchlorid, (Vergleichsbeispiel)
N-(3-(3-Ethoxy-4-methoxyphenyl)-1,4,5,6-tetrahydro-pyridazin-1-ylcarbonyl)-phenyl)-benzoyl-4-carboxamid, F. 186°;
mit 4-Chlorbenzoylchlorid,
N-(3-(3-Ethoxy-4-methoxyphenyl)-1,4,5,6-tetrahydro-pyridazin-1-ylcarbonyl)-phenyl)-4-chlorbenzoyl-4-carboxamid, F. 200°;
mit 3-Nitrobenzoylchlorid,
N-(3-(3-Ethoxy-4-methoxyphenyl)-1,4,5,6-tetrahydro-pyridazin-1-ylcarbonyl)-phenyl)-3-nitrobenzoyl-4-carboxamid, F. 233°.

### Beispiel 3:

Eine Suspension von 4,70 g 3-(3,4-Dimethoxyphenyl)-1,4,5,6-tetrahydropyridazin in 150 ml THF wird mit 2,24 g Kalium-tert.-butylat versetzt und 30 Minuten gerührt. Man gibt 5,44 g 3-Benzoylaminobenzoyl-chlorid dazu und rührt 10 Stunden bei Raumtemperatur nach. Das Lösungsmittel wird entfernt und es wird wie üblich aufgearbeitet. Man erhält N-(3-(3,4-Dimethoxyphenyl)-1,4,5,6-tetrahydropyridazin-1-ylcarbonyl)-phenyl)-3-benzoyl-3-carboxamid, F. 196°. (Vergleichsbeispiel)

Analog erhält man durch Umsetzung von 3-(3,4-Dimethoxyphenyl)-1,4,5,6-tetrahydropyridazin (Ausführungsbeispiele)
mit 3-(3,4-Dimethoxybenzoyl)-aminobenzoylchlorid,
N-(3-(3,4-Dimethoxyphenyl)-1,4,5,6-tetrahydro-pyridazin-1-ylcarbonyl)-phenyl)-3,4-dimethoxybenzoyl-3-carboxamid, F. 183°;
mit 3-(3-Methylbenzoyl)-aminobenzoylchlorid,
N-(3-(3,4-Dimethoxyphenyl)-1,4,5,6-tetrahydro-pyridazin-1-ylcarbonyl)-phenyl)-3-methylbenzoyl-3-carboxamid, F. 171 °;
mit 3-(3-Chlorbenzoyl)-aminobenzoylchlorid,
N-(3-(3,4-Dimethoxyphenyl)-1,4,5,6-tetrahydro-pyridazin-1-ylcarbonyl)-phenyl)-3-chlorbenzoyl-3-carboxamid, F. 172°;
mit 3-(4-Methoxybenzoyl)-aminobenzoylchlorid,
N-(3-(3,4-Dimethoxyphenyl)-1,4,5,6-tetrahydro-pyridazin-1-ylcarbonyl)-phenyl)-4-methoxybenzoyl-3-carboxamid, F. 203°.

### Beispiel 4:

Analog zu Beispiel 2 erhält man durch Umsetzung von 1-(4-Aminobenzoyl)-3-(3-isopropoxy-4-methoxyphenyl)-1,4,5,6-tetrahydro-pyridazin
mit 4-Butoxybenzoylchlorid,
N-(3-(3-Isopropoxy-4-methoxyphenyl)-1,4,5,6-tetrahydro-pyridazin-1-ylcarbonyl)-phenyl)-4-butoxybenzoyl-4-carboxamid, F. 161°;
mit 4-Ethoxybenzoylchlorid,
N-(3-(3-Isopropoxy-4-methoxyphenyl)-1,4,5,6-tetrahydro-pyridazin-1-ylcarbonyl)-phenyl)-4-ethoxybenzoyl-4-carboxamid, F. 171 °;
mit Benzoylchlorid, (Vergleichsbeispiel)
N-(3-(3-Isopropoxy-4-methoxyphenyl)-1,4,5,6-tetrahydro-pyridazin-1-ylcarbonyl)-phenyl)-benzoyl-4-carboxamid, F. 220°;
mit 3-Methylbenzoylchlorid,
N-(3-(3-Isopropoxy-4-methoxyphenyl)-1,4,5,6-tetrahydro-pyridazin-1-ylcarbonyl)-phenyl)-3-methylbenzoyl-4-carboxamid, F. 196°;
mit 4-Cyclopentyloxybenzoylchlorid,
N-(3-(3-Isopropoxy-4-methoxyphenyl)-1,4,5,6-tetrahydro-pyridazin-1-ylcarbonyl)-phenyl)-4-cyclopentyloxybenzoyl-4-carboxamid, F. 163°;
mit 4-Isopropoxybenzoylchlorid,
N-(3-(3-Isopropoxy-4-methoxyphenyl)-1,4,5,6-tetrahydro-pyridazin-1-ylcarbonyl)-phenyl)-4-isopropoxybenzoyl-4-carboxamid, F. 183°;
mit 4-Propoxybenzoylchlorid,
N-(3-(3-Isopropoxy-4-methoxyphenyl)-1,4,5,6-tetrahydro-pyridazin-1-ylcarbonyl)-phenyl)-4-propoxybenzoyl-4-carboxamid, F. 171°.

### Beispiel 5:

Analog zu Beispiel 1 erhält man durch Umsetzung von 1-(3-Aminobenzoyl)-3-(3-Cyclopentyloxy-4-methoxyphenyl)-1,4,5,6-tetrahydropyridazin
mit 3-Methylbenzoylchlorid,
N-(3-(3-Cyclopentyloxy-4-methoxyphenyl)-1,4,5,6-tetrahydropyridazin-1-ylcarbonyl)-phenyl)-3-methylbenzoyl-3-carboxamid, F. 144°;
mit 4-Methoxybenzoylchlorid,
N-(3-(3-Cyclopentyloxy-4-methoxyphenyl)-1,4,5,6-tetrahydropyridazin-1-ylcarbonyl)-phenyl)-4-methoxybenzoyl-3-carboxamid, F. 194°;
mit 4-Phenylbenzoylchlorid,
N-(3-(3-Cyclopentyloxy-4-methoxyphenyl)-1,4,5,6-tetrahydropyridazin-1-ylcarbonyl)-phenyl)-4-phenylbenzoyl-3-carboxamid, F. 140°.

### Beispiel 6:

Analog zu Beispiel 1 erhält man durch Umsetzung von 1-(3-Aminobenzoyl)-3-(3,4-diethoxyphenyl)-1,4,5,6-tetrahydro-pyridazin
mit 4-Chlorbenzoylchlorid,
N-(3-(3,4-Diethoxyphenyl)-1,4,5,6-tetrahydro-pyridazin-1-ylcarbonyl)-phenyl)-4-chlorbenzoyl-3-carboxamid, F. 152°;
mit 3-Nitrobenzoylchlorid,
N-(3-(3,4-Diethoxyphenyl)-1,4,5,6-tetrahydro-pyridazin-1-ylcarbonyl)-phenyl)-3-nitrobenzoyl-3-carboxamid, F. 105°;
mit 4-Butoxybenzoylchlorid,
N-(3-(3,4-Diethoxyphenyl)-1,4,5,6-tetrahydro-pyridazin-1-ylcarbonyl)-phenyl)-4-butoxybenzoyl-3-carboxamid, F. 103°;
mit 4-Ethoxybenzoylchlorid,
N-(3-(3,4-Diethoxyphenyl)-1,4,5,6-tetrahydro-pyridazin-1-ylcarbonyl)-phenyl)-4-ethoxybenzoyl-3-carboxamid, F. 181 °.

### Beispiel 7:

Analog zu Beispiel 2 erhält man durch Umsetzung von 1-(4-Aminobenzoyl)-3-(3,4-diethoxyphenyl)-1,4,5,6-tetrahydro-pyridazin
mit 4-Chlorbenzoylchlorid,
N-(3-(3,4-Diethoxyphenyl)-1 ,4,5 ,6-tetrahydro-pyridazin-1-ylcarbonyl)-phenyl)-4-chlorbenzoyl-4-carboxamid, F. 195°;
mit 3-Nitrobenzoylchlorid,
N-(3-(3,4-Diethoxyphenyl)-1,4,5,6-tetrahydro-pyridazin-1-ylcarbonyl)-phenyl)-3-nitrobenzoyl-4-carboxamid, F. 218°;
mit 4-Butoxybenzoylchlorid,
N-(3-(3,4-Diethoxyphenyl)-1,4,5,6-tetrahydro-pyridazin-1-ylcarbonyl)-phenyl)-4-butoxybenzoyl-4-carboxamid, F. 103°;
mit 4-Ethoxybenzoylchlorid,
N-(3-(3,4-Diethoxyphenyl)-1,4,5,6-tetrahydro-pyridazin-1-ylcarbonyl)-phenyl)-4-ethoxybenzoyl-4-carboxamid, F. 176°;
mit 4-Methoxybenzoylchlorid,
N-(3-(3,4-Diethoxyphenyl)-1,4,5,6-tetrahydro-pyridazin-1-ylcarbonyl)-phenyl)-4-methoxybenzoyl-4-carboxamid, F. 192°.

### Beispiel 8:

Analog zu Beispiel 1 erhält man durch Umsetzung von 1-(3-Aminobenzoyl)-3-(3-isopropoxy-4-methoxyphenyl)-1,4,5,6-tetrahydropyridazin
mit 4-Ethoxybenzoylchlorid,
N-(3-(3-Isopropoxy-4-methoxyphenyl)-1,4,5,6-tetrahydropyridazin-1-ylcarbonyl)-phenyl)-4-ethoxybenzoyl-3-carboxamid, F. 160°;
mit 4-Butoxybenzoylchlorid,
N-(3-(3-Isopropoxy-4-methoxyphenyl)-1,4,5,6-tetrahydropyridazin-1-ylcarbonyl)-phenyl)-4-butoxybenzoyl-3-carboxamid, F. 160°;
mit 4-Methoxybenzoylchlorid,
N-(3-(3-Isopropoxy-4-methoxyphenyl)-1,4,5,6-tetrahydropyridazin-1-ylcarbonyl)-phenyl)-4-methoxybenzoyl-3-carboxamid, F. 161°;
mit 4-Isopropoxybenzoylchlorid,
N-(3-(3-Isopropoxy-4-methoxyphenyl)-1,4,5,6-tetrahydropyridazin-1-ylcarbonyl)-phenyl)-4-isopropxybenzoyl-3-carboxamid, F. 168°;
mit 3-Nitrobenzoylchlorid,
N-(3-(3-Isopropoxy-4-methoxyphenyl)-1,4,5,6-tetrahydropyridazin-1-ylcarbonyl)-phenyl)-3-nitrobenzoyl-3-carboxamid, F. 194°.

### Beispiel 9:

Analog zu Beispiel 1 erhält man durch Umsetzung von 1-(3-Aminobenzoyl)-3-(3,4-dimethoxyphenyl)-1,4,5,6-tetrahydro-pyridazin
mit 4-Ethoxybenzoylchlorid,
N-(3-(3,4-Dimethoxyphenyl)-1,4,5,6-tetrahydro-pyridazin-1-ylcarbonyl)-phenyl)-4-ethoxybenzoyl-3-carboxamid, F. 176°;
mit 4-Butoxybenzoylchlorid,
N-(3-(3,4-Dimethoxyphenyl)-1,4,5,6-tetrahydro-pyridazin-1-ylcarbonyl)-phenyl)-4-butoxybenzoyl-3-carboxamid, F. 143°;
mit 4-Pentoxybenzoylchlorid,
N-(3-(3,4-Dimethoxyphenyl)-1,4,5,6-tetrahydro-pyridazin-1-ylcarbonyl)-phenyl)-4-pentoxybenzoyl-3-carboxamid, F. 140°;
mit 3-Propoxybenzoylchlorid,
N-(3-(3,4-Dimethoxyphenyl)-1,4,5,6-tetrahydro-pyridazin-1-ylcarbonyl)-phenyl)-3-propoxybenzoyl-3-carboxamid, F. 153°;
mit 4-Hexyloxybenzoylchlorid,
N-(3-(3,4-Dimethoxyphenyl)-1,4,5,6-tetrahydro-pyridazin-1-ylcarbonyl)-phenyl)-4-hexyloxybenzoyl-3-carboxamid, F. 162°;
mit 4-Decyloxybenzoylchlorid,
N-(3-(3,4-Dimethoxyphenyl)-1,4,5,6-tetrahydro-pyridazin-1-ylcarbonyl)-phenyl)-4-decyloxybenzoyl-3-carboxamid, F. 130°.

### Beispiel 10:

Analog zu Beispiel 1 erhält man durch Umsetzung von 1-(3-Aminobenzoyl)-3-(3-ethoxy-4-methoxyphenyl)-1,4,5,6-tetrahydro-pyridazin
mit 4-lsopropoxybenzoylchlorid,
N-(3-(3-Ethoxy-4-methoxyphenyl)-1,4,5,6-tetrahydro-pyridazin-1-ylcarbonyl)-phenyl)-4-isopropoxybenzoyl-3-carboxamid, F. 108°;
mit 3-Ethoxybenzoylchlorid,
N-(3-(3-Ethoxy-4-methoxyphenyl)-1,4,5,6-tetrahydro-pyridazin-1-ylcarbonyl)-phenyl)-3-ethoxybenzoyl-3-carboxamid, F. 142°;
mit 3-Butoxybenzoylchlorid,
N-(3-(3-Ethoxy-4-methoxyphenyl)-1,4,5,6-tetrahydro-pyridazin-1-ylcarbonyl)-phenyl)-3-butoxybenzoyl-3-carboxamid, F. 144°;
mit 3-Hexyloxybenzoylchlorid,
N-(3-(3-Ethoxy-4-methoxyphenyl)-1,4,5,6-tetrahydro-pyridazin-1-ylcarbonyl)-phenyl)-3-hexyloxybenzoyl-3-carboxamid, F. 137°;
mit 4-Decyloxybenzoylchlorid,
N-(3-(3-Ethoxy-4-methoxyphenyl)-1,4,5,6-tetrahydro-pyridazin-1-ylcarbonyl)-phenyl)-4-decyloxybenzoyl-3-carboxamid, F. 123°;
mit 3-Methoxycarbonylaminobenzoylchlorid,
N-(3-(3-Ethoxy-4-methoxyphenyl)-1,4,5,6-tetrahydro-pyridazin-1-ylcarbonyl)-phenyl)-3-methoxycarbonylaminobenzoyl-3-carboxamid, F. 193°;
mit 3-Ethoxycarbonylaminobenzoylchlorid,
N-(3-(3-Ethoxy-4-methoxyphenyl)-1,4,5,6-tetrahydro-pyridazin-1-ylcarbonyl)-phenyl)-3-ethoxycarbonylaminobenzoyl-3-carboxamid, F. 221°;
mit 3-Methansulfonylaminobenzoylchlorid,
N-(3-(3-Ethoxy-4-methoxyphenyl)-1,4,5,6-tetrahydro-pyridazin-1-ylcarbonyl)-phenyl)-3-methansulfonylaminobenzoyl-3-carboxamid, F. 174°;
mit 4-Methoxycarbonylaminobenzoylchlorid,
N-(3-(3-Ethoxy-4-methoxyphenyl)-1,4,5,6-tetrahydro-pyridazin-1-ylcarbonyl)-phenyl)-4-methoxycarbonylaminobenzoyl-3-carboxamid, F. 234°;
mit 4-Ethoxycarbonylaminobenzoylchlorid,
N-(3-(3-Ethoxy-4-methoxyphenyl)-1,4,5,6-tetrahydro-pyridazin-1-ylcarbonyl)-phenyl)-4-ethoxycarbonylaminobenzoyl-3-carboxamid, F. 221°;
mit 4-Acetylaminobenzoylchlorid,
N-(3-(3-Ethoxy-4-methoxyphenyl)-1,4,5,6-tetrahydro-pyridazin-1-ylcarbonyl)-phenyl)-4-acetylaminobenzoyl-3-carboxamid, F. 244°.

### Beispiel 11:

Analog zu Beispiel 2 erhält man durch Umsetzung von 1-(4-Aminobenzoyl)-3-(3-ethoxy-4-methoxyphenyl)-1,4,5,6-tetrahydro-pyridazin
mit 4-Acetylaminobenzoylchlorid,
N-(3-(3-Ethoxy-4-methoxyphenyl)-1,4,5,6-tetrahydro-pyridazin-1-ylcarbonyl)-phenyl)-4-acetylaminobenzoyl-4-carboxamid, F. >266°;
mit 4-Isopropylcarbonylaminobenzoylchlorid,
N-(3-(3-Ethoxy-4-methoxyphenyl)-1,4,5,6-tetrahydro-pyridazin-1-ylcarbonyl)-phenyl)-4-isopropylcarbonylaminobenzoyl-4-carboxamid, F. >260°;
mit 4-Methoxycarbonylaminobenzoylchlorid,
N-(3-(3-Ethoxy-4-methoxyphenyl)-1,4,5,6-tetrahydro-pyridazin-1-ylcarbonyl)-phenyl)-4-methoxycarbonylaminobenzoyl-4-carboxamid, F. 275°;
mit 4-Ethoxycarbonylaminobenzoylchlorid,
N-(3-(3-Ethoxy-4-methoxyphenyl)-1,4,5,6-tetrahydro-pyridazin-1-ylcarbonyl)-phenyl)-4-ethoxycarbonylaminobenzoyl-4-carboxamid, F. 246°;
mit 4-Methansulfonylaminobenzoylchlorid,
N-(3-(3-Ethoxy-4-methoxyphenyl)-1,4,5,6-tetrahydro-pyridazin-1-ylcarbonyl)-phenyl)-4-methansulfonylaminobenzoyl-4-carboxamid, F. >260°.

### Beispiel 12:

Analog zu Beispiel 1 erhält man durch Umsetzung von 1-(3-Aminobenzoyl)-3-(3,4-diethoxyphenyl)-1,4,5,6-tetrahydro-pyridazin
mit 4-Pentoxybenzoylchlorid,
N-(3-(3,4-Diethoxyphenyl)-1,4,5,6-tetrahydro-pyridazin-1 - ylcarbonyl)-phenyl)-4-pentoxybenzoyl-3-carboxamid, F. 145°;
mit 3-Propoxybenzoylchlorid,
N-(3-(3,4-Diethoxyphenyl)-1,4,5,6-tetrahydro-pyridazin-1-ylcarbonyl)-phenyl)-3-propoxybenzoyl-3-carboxamid, F. 112°;
mit 3-Butoxybenzoylchlorid,
N-(3-(3,4-Diethoxyphenyl)-1,4,5,6-tetrahydro-pyridazin-1-ylcarbonyl)-phenyl)-3-butoxybenzoyl-3-carboxamid, F. 120°;
mit 3-Hexyloxybenzoylchlorid,
N-(3-(3,4-Diethoxyphenyl)-1,4,5,6-tetrahydro-pyridazin-1-ylcarbonyl)-phenyl)-3-hexyloxybenzoyl-3-carboxamid, F. 151 °;
mit 3-Ethoxybenzoylchlorid,
N-(3-(3,4-Diethoxyphenyl)-1,4,5,6-tetrahydro-pyridazin-1-ylcarbonyl)-phenyl)-3-ethoxybenzoyl-3-carboxamid, F. 141 °.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 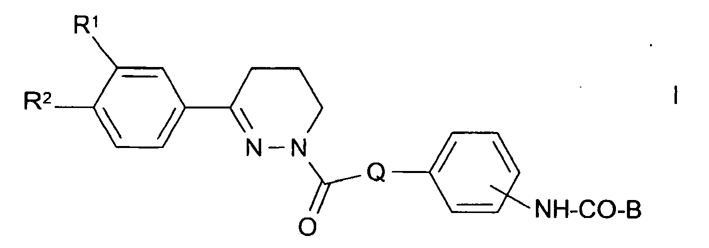 worin
B o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-N,N-Dimethylaminophenyl, o-, m- oder p-Nitrophenyl, o-, m- oder p-Hydroxyphenyl, o-, m- oder p-Methoxyphenyl, o-, m- oder p-Ethoxyphenyl, o-, m- oder p-Isopropoxyphenyl, o-, m- oder p-Butoxyphenyl, o-, m- oder p-Pentoxyphenyl, o-, m- oder p-Hexyloxyphenyl, o-, m- oder p-Decyloxyphenyl, o-, m-, p-Trifluormethylphenyl, o-, m- oder p-Fluorphenyl, o-, m- oder p-Chlorphenyl, o-, m- oder p-Bromphenyl, o-, m- oder p-Acetylaminophenyl, o-, m- oder p-Isopropylcarbonylaminophenyl, o-, m- oder p-Methansulfonylaminophenyl, o-, m- oder p-Ethansulfonyl-aminophenyl, o-, m- oder p-Methoxycarbonyl-aminophenyl, o-, m- oder p-Ethoxycarbonyl-aminophenyl, 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dimethoxyphenyl,
Q fehlt,
R¹, R ² jeweils unabhängig voneinander -OR⁴,
R⁴ A, Cycloalkyl mit 3-7 C-Atomen,
A Alkyl mit 1 bis 10 C-Atomen, das durch 1 bis 5 F- und/oder Cl-Atome substituiert sein kann und
Hal F, Cl, Br oder I
bedeuten,
sowie deren physiologisch unbedenklichen Salze und Solvate.

2. Verbindungen der Formel I gemäß Anspruch 1
(a) N-(3-(3-Ethoxy-4-methoxyphenyl)-1,4,5,6-tetrahydropyridazin-1-ylcarbonyl)-phenyl)-4-methoxybenzoyl-4-carboxamid;
(b) N-(3-(3-Ethoxy-4-methoxyphenyl)-1,4,5,6-tetrahydropyridazin-1-ylcarbonyl)phenyl)-4-fluorobenzoyl-4-carboxamid;
(c) N-(3-(3-Ethoxy-4-methoxyphenyl)-1,4,5,6-tetrahydropyridazin-1-ylcarbonyl)-phenyl)-4-chlorbenzoyl-4-carboxamid;
(d) N-(3-(3-Ethoxy-4-methoxyphenyl)-1,4,5,6-tetrahydropyridazin-1-ylcarbonyl)phenyl)-3-nitrobenzoyl-4-carboxamid;
(e) N-3-3-Ethoxy-4-methoxyphenyl)-1,4,5,6-tetrahydropyridazin-1-ylcarbonyl)phenyl)-4-acetylaminobenzoyl-4-carboxamid;
(f) N-3-3-Ethoxy-4-methoxyphenyl)-1,4,5,6-tetrahydropyridazin-1-ylcarbonyl)phenyl)-4-methoxycarbonylaminobenzoyl-4-carboxamid;
(g) N-3-3-Ethoxy-4-methoxyphenyl)-1,4,5,6-tetrahydropyridazin-1-ylcarbonyl)phenyl)-4-ethoxycarbonylaminobenzoyl-4-carboxamid; sowie deren physiologisch unbedenklichen Salze und Solvate.

3. Verbindung gemäß Anspruch 1 oder 2, **dadurch gekennzeichnet, dass** die Verbindung der Formel I a) N-(3-(3-Ethoxy-4-methoxyphenyl)-1,4,5,6-tetrahydropyridazin-1-ylcarbonyl)-phenyl)-4-methoxybenzoyl-4-carboxamid ist.

4. Verfahren zur Herstellung von Verbindungen der Formel I nach Anspruch 1 sowie deren Salzen, **dadurch gekennzeichnet, dass** man eine Verbindung der Formel II 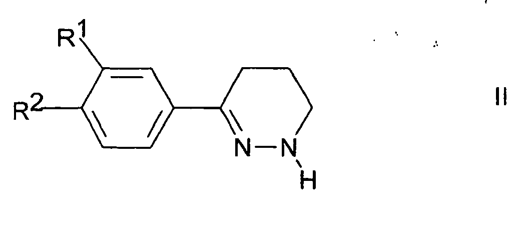 worin
R¹ und R² die angegebenen Bedeutungen haben,
mit einer Verbindung der Formel III 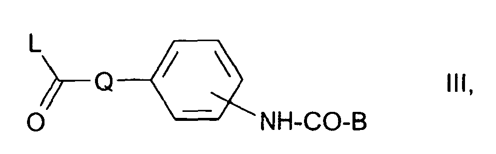 worin
B und Q die angegebenen Bedeutungen haben, und
L Cl, Br, OH oder eine reaktionsfähige veresterte OH-Gruppe bedeutet,
umsetzt,
oder
dass man eine Verbindung der Formel IV 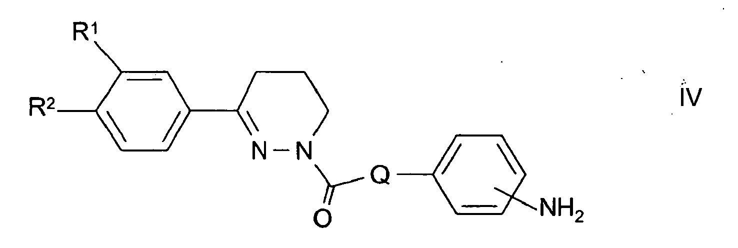 worin
R¹, R² und Q die angegebenen Bedeutungen haben, mit einer Verbindung der Formel V
B-CO-L V
worin
B die angegebene Bedeutung hat, und
L Cl, Br, OH oder eine reaktionsfähige veresterte OH-Gruppe bedeutet,
umsetzt,
und/oder dass man eine basische Verbindung der Formel I durch Behandeln mit einer Säure in eines ihrer Salze überführt.

5. Verbindungen der Formel I nach Anspruch 1 und ihre physiologisch unbedenklichen Salze und Solvate als Arzneimittel.

6. Verbindungen der Formel I nach Anspruch 1 und ihre physiologisch unbedenklichen Salze und Solvate als Phosphodiesterase IV-Hemmer.

7. Pharmazeutische Zubereitung, **gekennzeichnet durch** einen Gehalt an mindestens einer Verbindung der Formel I nach Anspruch 1 und/oder einem ihrer physiologisch unbedenklichen Salze und/oder eines ihrer Solvate.

8. Verfahren zur Herstellung pharmazeutischer Zubereitungen, **dadurch gekennzeichnet, dass** man eine Verbindung der Formel I nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

9. Verbindungen der Formel I nach Anspruch 1 und ihre physiologisch unbedenklichen Salze und Solvate zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

10. Verwendung von Verbindungen der Formel I nach Anspruch 1 und/oder ihre physiologisch unbedenklichen Salze oder Solvate zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

11. Verwendung von Verbindungen der Formel I nach Anspruch 1 und/oder ihrer physiologisch unbedenklichen Salze und/oder Solvate zur Herstellung eines Arzneimittels zur Bekämpfung von Krankheiten.

## Claims

1. Compounds of formula I: 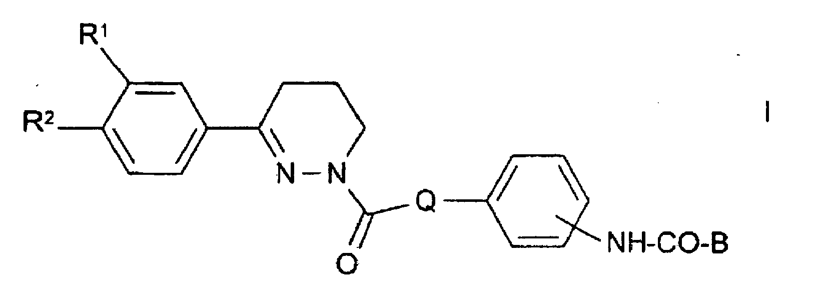 in which
B is o-, m- or p-methylphenyl, o-, m- or p-ethylphenyl, o-, m- or p-propylphenyl, o-, m- or p-isopropylphenyl, o-, m- or p-tert-butylphenyl, o-, m- or p-N,N-dimethylaminophenyl, o-, m- or p-nitrophenyl, o-, m- or p-hydroxyphenyl, o-, m- or p-methoxyphenyl, o-, m- or p-ethoxyphenyl, o-, m-or p-isopropoxyphenyl, o-, m- or p-butoxyphenyl, o-, m- or p-pentoxyphenyl, o-, m- or p-hexyloxyphenyl, o-, m- or p-decyloxyphenyl, o-, m- or p-trifluoromethylphenyl, o-, m- or p-fluorophenyl, o-, m- or p-chlorophenyl, o-, m- or p-bromophenyl, o-, m- or p-acetylaminophenyl, o-, m- or p-isopropylcarbonylaminophenyl, o-, m- or p-methanesulfonylaminophenyl, o-, m- or p-ethanesulfonylaminophenyl, o-, m- or p-methoxycarbonylaminophenyl, o-, m- or p-ethoxycarbonylaminophenyl, 2,3-, 2,4-, 2,5-, 2,6-, 3,4- or 3,5-dimethylphenyl, 2,3-, 2,4-, 2,5-, 2,6-, 3,4- or 3,5-dihydroxyphenyl, 2,3-, 2,4-, 2,5-, 2,6-, 3,4- or 3,5-difluorophenyl, 2,3-, 2,4-, 2,5-, 2,6-, 3,4-or 3,5-dichlorophenyl or 2,3-, 2,4-, 2,5-, 2,6-, 3,4- or 3,5-dimethoxyphenyl,
Q is absent,
R¹, R² each independently of one another are -OR⁴,
R⁴ is A or cycloalkyl having 3-7 C atoms,
A is alkyl having 1 to 10 C atoms which can be substituted by 1 to 5 F and/or Cl atoms, and
Hal is F, Cl, Br or I,
and their physiologically acceptable salts and solvates.

2. Compounds of formula I according to Claim 1:
(a) N-(3-(3-ethoxy-4-methoxyphenyl)-1,4,5,6-tetrahydropyridazin-1-ylcarbonylphenyl)-4-methoxybenzoyl-4-carboxamide;
(b) N-(3-(3-ethoxy-4-methoxyphenyl)-1,4,5,6-tetrahydropyridazin-1-ylcarbonylphenyl)-4-fluorobenzoyl-4-carboxamide;
(c) N-(3-(3-ethoxy-4-methoxyphenyl)-1,4,5,6-tetrahydropyridazin-1-ylcarbonylphenyl)-4-chlorobenzoyl-4-carboxamide;
(d) N-(3-(3-ethoxy-4-methoxyphenyl)-1,4,5,6-tetrahydropyridazin-1-ylcarbonylphenyl)-3-nitrobenzoyl-4-carboxamide;
(e) N-(3-(3-ethoxy-4-methoxyphenyl)-1,4,5,6-tetrahydropyridazin-1-ylcarbonylphenyl)-4-acetylaminobenzoyl-4-carboxamide;
(f) N-(3-(3-ethoxy-4-methoxyphenyl)-1,4,5,6-tetrahydropyridazin-1-ylcarbonylphenyl)-4-methoxycarbonylaminobenzoyl-4-carboxamide;
(g) N-(3-(3-ethoxy-4-methoxyphenyl)-1,4,5,6-tetrahydropyridazin-1-ylcarbonylphenyl)-4-ethoxycarbonylaminobenzoyl-4-carboxamide; and their physiologically acceptable salts and solvates.

3. Compound according to Claim 1 or 2, **characterized in that** the compound of formula I a) is N-(3-(3-ethoxy-4-methoxyphenyl)-1,4,5,6-tetrahydropyridazin-1-ylcarbonylphenyl)-4-methoxybenzoyl-4-carboxamide.

4. Process for the preparation of compounds of formula I according to Claim 1 and their salts, **characterized in that** a compound of formula II: 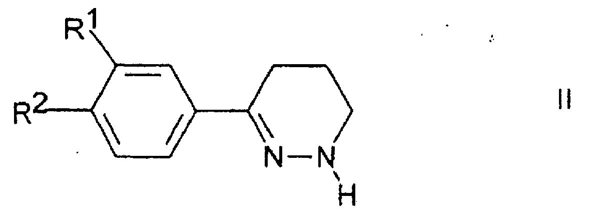 in which
R¹ and R² are as defined,
is reacted with a compound of formula III: 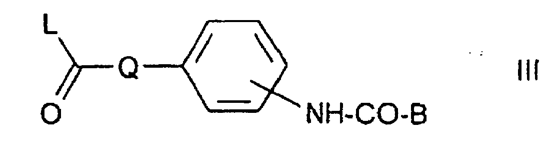 in which
B and Q are as defined, and
L is Cl, Br, OH or a reactive esterified OH group,
or
**in that** a compound of formula IV: 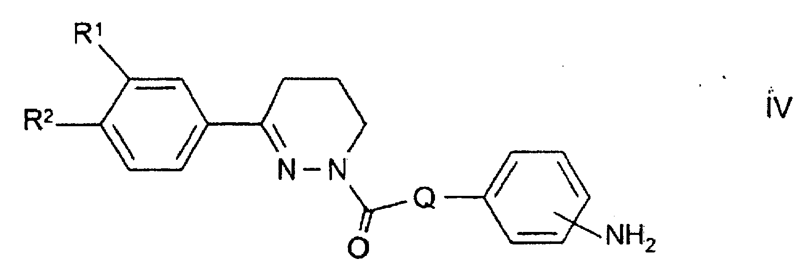 in which
R¹, R² and Q are as defined,
is reacted with a compound of formula V:
B-CO-L V
in which
B is as defined, and
L is Cl, Br, OH or a reactive esterified OH group,
and/or **in that** a basic compound of formula I is converted to one of its salts by treatment with an acid.

5. Compounds of formula I according to Claim 1 and their physiologically acceptable salts and solvates as drugs.

6. Compounds of formula I according to Claim 1 and their physiologically acceptable salts and solvates as phosphodiesterase IV inhibitors.

7. Pharmaceutical formulation, **characterized in that** it contains at least one compound of formula I according to Claim 1 and/or one of their physiologically acceptable salts and/or one of their solvates.

8. Process for the preparation of pharmaceutical formulations, **characterized in that** a compound of formula I according to Claim 1 and/or one of its physiologically acceptable salts and/or one of its solvates are converted to a suitable dosage form together with at least one solid, liquid or semiliquid excipient or auxiliary substance.

9. Compounds of formula I according to Claim 1 and their physiologically acceptable salts and solvates for combatting allergic diseases, asthma, chronic bronchitis, atopic dermatitis, psoriasis and other skin diseases, inflammatory diseases, autoimmune diseases, e.g. rheumatoid arthritis, multiple sclerosis, Crohn's disease, diabetes mellitus or ulcerative colitis, osteoporosis, transplant rejection reactions, cachexia, tumoural growth or tumoural metastases, sepsis, memory disorders, atherosclerosis and AIDS.

10. Use of compounds of formula I according to Claim 1 and/or their physiologically acceptable salts or solvates for the preparation of a drug for combatting allergic diseases, asthma, chronic bronchitis, atopic dermatitis, psoriasis and other skin diseases, inflammatory diseases, autoimmune diseases, e.g. rheumatoid arthritis, multiple sclerosis, Crohn's disease, diabetes mellitus or ulcerative colitis, osteoporosis, transplant rejection reactions, cachexia, tumoural growth or tumoural metastases, sepsis, memory disorders, atherosclerosis and AIDS.

11. Use of compounds of formula I according to Claim 1 and/or their physiologically acceptable salts and/or solvates for the preparation of a drug for combatting diseases.

## Revendications

1. Composés de la formule I 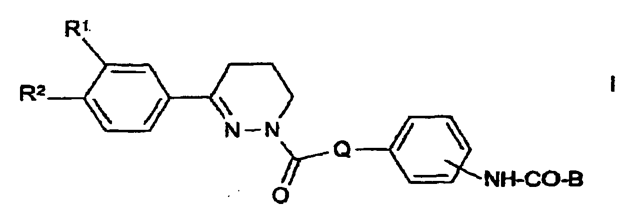 dans laquelle
B signifie o-, m- ou p-méthylphényle, o-, m- ou p-éthylphényle, o-, m- ou p-propylphényle, o-, m- ou p-isopropylphényle, o-, m- ou p-tert.-butylphényle, o-, m- ou p-N,N-diméthylaminophényle, o-, m- ou p-nitrophényle, o-, m- ou p-hydroxyphényle, o-, m- ou p-méthoxyphényle, o-, m- ou p-éthoxyphényle, o-, m- ou p-isopropoxyphényle, o-, m- ou p-butoxyphényle, o- m- ou p-pentoxyphényle, o-, m- ou p-hexyloxyphényle, o-, mou p-décyloxyphényle, o-, m-, p-trifluorométhylphényle, o-, m- ou p-fluorophényle, o-, m- ou p-chlorophényle, o-, m- ou p-bromophényle, o-, m- ou p-acétylaminophényle, O-, m- ou p-isopropylcarbonylaminophényle, O-, m- ou p-méthansulfonylaminophényle, o-, m- ou p-éthansulfonylaminophényle, o-, m- ou p-méthoxycarbonyl-aminophényle, o-, m- ou p-éthoxycarbonyl-aminophényle, 2,3-, 2,4-, 2,5-, 2,6-, 3,4- ou 3,5-diméthylphényle, 2,3-, 2,4-, 2,5-, 2,6-, 3,4- ou 3,5-dihydroxyphényle, 2,3-, 2,4-, 2,5-, 2,6-, 3,4- ou 3,5-difluorophényle, 2,3-, 2,4-, 2,5-, 2,6-, 3,4- ou 3,5-dichlorophényle, 2,3-, 2,4-, 2,5-, 2,6-, 3,4- ou 3,5-diméthoxyphényle,
Q manque,
R¹, R² signifient respectivement indépendamment l'un de l'autre -OR⁴,
R⁴ signifie A, un cycloalkyle avec 3 à 7 atomes de carbone,
A signifie un alkyle avec 1 à 10 atomes de carbone, qui peut être substitué par 1 à 5 atomes de F et/ou Cl et
Hal signifie F, Cl, Br ou I,
ainsi que leurs sels et solvates physiologiquement acceptables.

2. Composés de la formule I selon la revendication 1
(a) N-(3-(3-éthoxy-4-méthoxyphényl)-1,4,5,6-tétrahydropyridazin-1-ylcarbonyl)-phényl)-4-méthoxybenzoyl-4-carboxamide,
(b) N-(3-(3-éthoxy-4-méthoxyphényl)-1,4,5,6-tétrahydropyridazin-1-ylcarbonyl)phényl)-4-fluorobenzoyl-4-carboxamid ;
(c) N-(3-(3-éthoxy-4-méthoxyphényl)-1,4,5,6-tétrahydropyridazin-1-ylcarbonyl)-phényl)-4-chlorobenzoyl-4-carboxamid ;
(d) N-(3-(3-éthoxy-4-méthoxyphényl)-1,4,5,6-tétrahydropyridazin-1-ylcarbonyl)phényl)-3-nitrobenzoyl-4-carboxamid ;
(e) N-3-3-éthoxy-4-méthoxyphényl)-1,4,5,6-tétrahydropyridazin-1-ylcarbonyl)phényl)-4-acétylaminobenzoyl-4-carboxamid ;
(f) N-3-3-éthoxy-4-méthoxyphényl)-1,4,5,6-tétrahydropyridazin-1-ylcarbonyl)phényl)-4-méthoxycarbonylaminobenzoyl-4-carboxamid ;
(g) N-3-3-éthoxy-4-méthoxyphényl)-1,4,5,6 tétrahydropyridazin-1-ylcarbonyl)phényl)-4-éthoxycarbonylaminobenzoyl-4-carboxamid ;
ainsi que leurs sels et solvates physiologiquement acceptables.

3. Composé selon la revendication 1 ou 2, **caractérisé en ce que** le composé de la formule I a) est un N-(3-(3-éthoxy-4-méthoxyphényl)-1,4,5,6-tétrahydropyridazin-1-ylcarbonyl)-phényl)-4-méthoxybenzoyl-4-carboxamid.

4. Procédé de préparation des composés de la formule I selon la revendication 1 ainsi que de leurs sels **caractérisé en ce que** l'on fait réagir un composé de la formule II 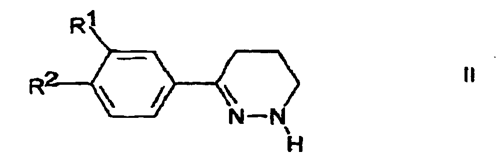 dans laquelle
R¹ et R² ont les significations données,
avec un composé de la formule III 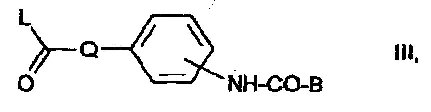 dans laquelle
B et Q ont les significations données, et
L signifie Cl, Br, OH ou un groupe OH estérifié réactif,
ou
**en ce que** l'on fait réagir un composé de la formule IV 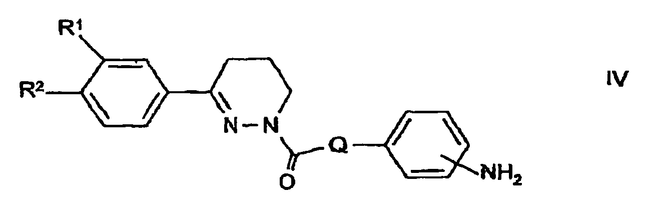 dans laquelle
R¹, R² et Q ont les significations données, avec
un composé de la formule V
B-CO-L V
dans laquelle
B a la signification donnée, et
L signifie Cl, Br, OH ou un groupe OH estérifié réactif,
et/ou **en ce qu'**on transforme un composé basique de la formule I en un de ses sels en le traitant par un acide.

5. Composés de la formule I selon la revendication 1 et leurs sels et solvates physiologiquement acceptables comme médicaments.

6. Composés de la formule I selon la revendication 1 et leurs sels et solvates physiologiquement acceptables comme inhibiteurs de phosphodiestérase IV.

7. Préparation pharmaceutique, **caractérisée par** une teneur en au moins un composé de la formule I selon la revendication 1 et/ou un de ses sels physiologiquement acceptables et/ou un de ses solvates.

8. Procédé de production de préparations pharmaceutiques, **caractérisé en ce que** l'on un met ensemble un composé de la formule I selon la revendication 1 et/ou un de ses sels physiologiquement acceptables et/ou de ses solvates avec au moins une substance porteuse ou auxiliaire solide, liquide ou semi-liquide dans une forme de dosage appropriée.

9. Composés de la formule I selon la revendication 1 et leurs sels et solvates physiologiquement acceptables pour la lutte contre les affections allergiques, l'asthme, la bronchite chronique, la dermatite atopique, le psoriasis et d'autres dermatoses, des maladies inflammatoires, des maladies auto-immunes comme par exemple l'arthrite rhumatoïde, la sclérose en plaque, la maladie de Crohn, le diabète sucré ou la colite ulcérante, l'ostéoporose, les réactions de rejet de transplant, la cachexie, la croissance tumorale ou les métastases tumorales, la septicémie, les troubles de la mémoire, l'athérosclérose et le SIDA.

10. Utilisation de composés de la formule I selon la revendication 1 et/ou de leurs sels et solvates physiologiquement acceptables à la préparation d'un médicament pour la lutte contre les affections allergiques, l'asthme, la bronchite chronique, la dermatite atopique, le psoriasis et d'autres dermatoses, des maladies inflammatoires, des maladies auto-immunes comme par exemple l'arthrite rhumatoïde, la sclérose en plaque, la maladie de Crohn, le diabète sucré ou la colite ulcérante, l'ostéoporose, les réactions de rejet de transplant, la cachexie, la croissance tumorale ou les métastases tumorales, la septicémie, les troubles de la mémoire, l'athérosclérose et le SIDA.

11. Utilisation de composés de la formule I selon la revendication 1 et/ou de leurs sels et solvates physiologiquement acceptables à la préparation d'un médicament pour la lutte contre les maladies.
